# EUROPEAN PATENT SPECIFICATION

(11) **EP 4 512 464 B1**
(45) Date of publication and mention of the grant of the patent: **08.04.2026**
(21) Application number: 23854981.0
(22) Date of filing: 19.05.2023
(51) Int. Cl.: A61N 1/36, A61N 1/04, A61B 5/024, A61B 5/346, A61B 5/374, A61B 5/00, G16H 20/30

(54) **ELECTRICAL STIMULATION APPLICATION APPARATUS FOR PNEUMOGASTRIC NERVES REFLECTING DEGREE OF SYMPTOMS ACCORDING TO BIOSIGNAL MEASUREMENT**
ELEKTRISCHE STIMULATIONSANWENDUNGSVORRICHTUNG FÜR PNEUMOGASTRISCHE NERVEN ZUR REFLEXION DES SYMPTOMGRADES GEMÄSS BIOSIGNALMESSUNG
APPAREIL D'APPLICATION DE STIMULATION ÉLECTRIQUE POUR NERFS PNEUMOGASTRIQUES REFLÉTANT LE DEGRÉ DE SYMPTÔMES SELON UNE MESURE DE BIOSIGNAL

(30) Priority: 17.08.2022 KR 20220102862; 17.08.2022 KR 20220102864
(43) Date of publication of application: 26.02.2025
(73) Proprietor: Neurive Co., Ltd., Juchon-myeon Gimhae-si, Gyeongsangnam-do 50969 (KR)
(72) Inventor: SONG, Jae Jun, Seoul 06501 (KR); CHOI, Hyuk, Seoul 06095 (KR); HONG, Ki Hwan, Seoul 03709 (KR); MOON, Byoung Jong, Siheung-si, Gyeonggi-do 14922 (KR)
(74) Representative: Franke, Dirk
(86) International application number: PCT/KR2023/006863
(87) International publication number: WO 2024/039008

(56) References cited:
- WO-A1-2020/185601
- JP-A- H10 108 913
- JP-B2- 6 740 315
- KR-A- 20180 107 750
- KR-A- 20210 154 677
- KR-B1- 101 438 299
- US-A1- 2007 260 285
- US-A1- 2017 203 103
- US-A1- 2021 283 400

## Description

### [Technical Field]

The present disclosure relates to an apparatus for applying electrical stimulation to a pneumogastric nerve and, in more detail, an apparatus for applying electrical stimulation to a pneumogastric nerve through an electrode that is in contact with the skin of a human body.

### [Related Art]

The pneumogastric nerve is one of cranial nerves and corresponds to the tenth cranial nerve. The pneumogastric nerve is a mixed nerve coming out from the brain, distributed through the face, chest, and abdomen, and including parasympathetic nerve fibers, and takes part in controlling of parasympathetic nerves acting on the heart, lungs, alimentary canal, etc. The pneumogastric nerve has the longest and the most complicated structure of the cranial nerves and includes all of the sensory nerve fibers and the motor nerve fibers.

Pneumogastric nerve stimulation devices for the treatment of epilepsy and depression have reportedly received approval from the U.S. Food and Drug Administration (FDA) and these devices target the cervical branches positioned in the neck. However, such electrical stimulation of the cervical branches of a pneumogastric nerve involves a procedure of making a direct incision in the skin, exposing the cervical branches of the pneumogastric nerve, winding a coil, which is an electrolyte, around it, and implanting a microchip. Accordingly, it cannot be used in the realm of self-treatment by the general public. Patent application publication WO 2020/185601 A1 discloses an electrical stimulation application apparatus for pneumogastric nerves, wherein the stimulation is based on brain waves or heart rate variability.

Meanwhile, eastern medicine applies acupuncture on the ear as a method to treat diseases, the ear is an area where auricular branch of the pneumogastric nerve is distributed, and it can be considered that stimulation of the pneumogastric nerve is utilized for treatment.

Accordingly, devices that electrically stimulate a pneumogastric nerve distributed in the ear have been developed for the purposes of alleviating symptoms, etc., but it is difficult to accurately reflect the current state of users, so such devices cannot obtain sufficient effects, and for this reason, such devices are not widely used.

### [Disclosure]

### [Technical Problem]

The present disclosure has been made in an effort to solve the problems of the related art described above and an objective of the present disclosure is to provide a an apparatus for applying electrical stimulation to a pneumogastric nerve, thereby having an improved effect by reflecting the current degree of symptom of a user.

### [Technical Solution]

The present invention is defined by the appended claims. Aspects, embodiments and examples described hereinafter are only of exemplary nature and are presented for better understanding the present invention.
In order to achieve the objectives, an electrical stimulation application apparatus as specified in claim 1 is disclosed.

The biosignal that is measured may be a brain wave or heart rate variability.

When the biosignal that is measured is a brain wave, an FSWR may be calculated for each of a plurality of times of measurement over the predetermined intervals and an FSWRₛ that is an FSWR reference value may be set in the reference value setting step; an FSWRₜ calculated by measuring a brain wave of the user may be compared with the FSWRₛ in the stimulation value adjustment step; and the FSWR may be an index reflecting a state of a symptom of the user and may be calculated by dividing power of a fast wave band with a relatively high frequency by power of a slow wave band with a relatively low frequency in the measured brain wave.

The fast wave band may be a brain wave exceeding 13Hz and the slow wave band may be a brain wave of 13Hz or less.

In the reference value setting step, the FSWRₛ may be obtained by adding standard deviation of FSWR values calculated by measuring a brain wave a plurality of times to an average of the FSWR values.

In the stimulation value adjustment step, when the FSWRₜ is larger than the FSWRₛ, electrical stimulation that is applied through the electrode may be adjusted.

When the FSWRₜ is larger than the FSWRₛ, a duty ratio of electrical stimulation may be increased, and the duty ratio may be increased in proportion to a difference between the FSWRₜ and the FSWRₛ.

When the biosignal that is measured is heart rate variability, an FSBR may be calculated for each of a plurality of times of measurement over the predetermined intervals and an FSBRₛ that is an FSBR reference value may be set in the reference value setting step; an FSBRₜ calculated by measuring heart rate variability of the user may be compared with the FSBRₛ in the stimulation value adjustment step; and the FSBR may be an index reflecting a state of a symptom of the user and may be calculated by dividing power of a high frequency band with a relatively high frequency by power of a low frequency band with a relatively low frequency in the measured heart rate variability.

The high frequency band may be the range of 0.15Hz or more and 0.4Hz or less and the low frequency band may be the range of 0.04Hz or more and less than 0.15Hz.

In the reference value setting step, the FSBRₛ may be obtained by adding standard deviation of FSBR values calculated by measuring heart rate variability a plurality of times to an average of the FSBR values.

In the stimulation value adjustment step, when the FSBRₜ is larger than the FSBRₛ, electrical stimulation that is applied through the electrode may be adjusted.

When the FSBRₜ is larger than the FSBRₛ, a duty ratio of electrical stimulation may be increased, and the duty ratio may be increased in proportion to a difference between the FSBRₜ and the FSBRₛ.

An initial stimulation value setting step of deriving initial stimulation values adjusting numerical values included in an electrical stimulation guideline by applying an SPI calculated by applying one or more of demographic markers and environmental markers may be performed before the stimulation value adjustment step; the stimulation value adjustment step may adjust the initial stimulation values; and the SPI may be an index quantifying sensitivity to electrical stimulation, the demographic markers may be markers statistically reflecting sensitivity to electrical stimulation, and the environmental markers may be markers reflecting environmental factors influencing sensitivity to electrical stimulation.

The demographic markers may include one or more pieces of information of age, sex, BMI, presence of medication, and presence of disease.

The environmental markers may include one or more pieces of information of temperature, humidity, illuminance, and a discomfort index.

An electrical stimulation application apparatus for pneumogastric nerves according to another aspect of the present disclosure includes: an electrode configured to apply electrical stimulation in contact with the skin of a human body; an input unit configured to receive an electrical stimulation guideline comprising numerical information about electrical stimulation; a reference value setter configured to set a reference value for a biosignal of a user from the biosignal measured a plurality of times over predetermined intervals without electrical stimulation applied; and a stimulation value adjuster configured to adjust the electrical stimulation guideline by comparing a value calculated by measuring the biosignal of the user with the reference value before applying electrical stimulation.

The biosignal that is measured may be a brain wave or heart rate variability.

When the biosignal that is measured is a brain wave, the reference value setter may calculate an FSWR from each of brain wave information of the user measured a plurality of times over the predetermined intervals and may set an FSWRₛ that is an FSWR reference value, the stimulation value adjuster may adjust the electrical stimulation guideline by comparing an FSWRₜ calculated by measuring a brain wave of the user with the FSWRₛ before applying electrical stimulation, and the FSWR may be an index reflecting a state of a symptom of the user and may be calculated by dividing power of a fast wave band with a relatively high frequency by power of a slow wave band with a relatively low frequency in the measured brain wave.

The fast wave band may be a brain wave exceeding 13Hz and the slow wave band may be a brain wave of 13Hz or less.

The FSWRₛ may be obtained by adding standard deviation of FSWR values calculated by measuring a brain wave a plurality of times to an average of the FSWR values.

When the FSWRₜ is larger than the FSWRₛ, the stimulation value adjuster may adjust electrical stimulation that is applied through the electrode.

When the FSWRₜ is larger than the FSWRₛ, a duty ratio of electrical stimulation may be increased, and the duty ratio may be increased in proportion to a difference between the FSWRₜ and the FSWRₛ.

When the biosignal that is measured is heart rate variability, the reference value setter may calculate an FSBR from each of heart rate variability information of the user measured a plurality of times over the predetermined intervals and may set an FSBRₛ that is an FSBR reference value, the stimulation value adjuster may adjust the electrical stimulation guideline by comparing an FSBRₜ calculated by measuring heart rate variability of the user with the FSBRₛ before applying electrical stimulation, and the FSBR may be an index reflecting a state of a symptom of the user and may be calculated by dividing power of a high frequency band with a relatively high frequency by power of a low frequency band with a relatively low frequency in the measured heart rate variability.

The high frequency band may be the range of 0.15Hz or more and 0.4Hz or less and the low frequency band may be the range of 0.04Hz or more and less than 0.15Hz.

The FSBRₛ may be obtained by adding standard deviation of FSBR values calculated by measuring heart rate variability a plurality of times to an average of the FSBR values.

When the FSBRₜ is larger than the FSBRₛ, the stimulation value adjuster may adjust electrical stimulation that is applied through the electrode.

When the FSBRₜ is larger than the FSBRₛ, a duty ratio of electrical stimulation may be increased, and the duty ratio may be increased in proportion to a difference between the FSBRₜ and the FSBRₛ.

The electrical stimulation application apparatus may further include an initial stimulation value setter configured to derive initial stimulation values adjusting numerical values included in an electrical stimulation guideline by applying an SPI calculated by applying one or more of demographic markers and environmental markers; the SPI may be an index quantifying sensitivity to electrical stimulation, the demographic markers may be markers statistically reflecting sensitivity to electrical stimulation, and the environmental markers may be markers reflecting environmental factors influencing sensitivity to electrical stimulation; and the stimulation value adjuster may adjust the initial stimulation values derived by the initial stimulation value setter.

The demographic markers may include one or more pieces of information of age, sex, BMI, presence of medication, and presence of disease.

The environmental markers may include one or more pieces of information of temperature, humidity, illuminance, and a discomfort index.

### [Advantageous Effects]

The present disclosure configured as described above has an effect of further improving the effect by electrical stimulation by applying electrical stimulation while reflecting the current state of a user.

Further, since electrical stimulation is applied while sensitivity to electrical stimulation is reflected, there is an effect of being able to prevent the problem that a user feels pain due to electrical stimulation even when adjusting to the current state.

### [Brief Description of Drawings]

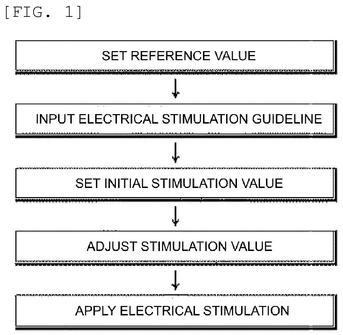

FIG. 1 is a flowchart illustrating an electrical stimulation application exemplary method for pneumogastric nerves reflecting degree of symptoms according to biosignal measurement.

### [Mode for Invention]

Embodiments of the present disclosure are described in detail with reference to the accompanying drawings.

The shape, sizes, etc. of elements may be exaggerated in the drawings for clearer description and elements indicated by the same reference numerals in the drawings are the same components.

Further, when an element is referred to as being "connected with" another element throughout the specification, it may be "directly connected" to the other element and may also be "electrically connected" to the other element with another element intervening therebetween. Further, unless explicitly described otherwise, "comprising" or "having" any components will be understood to imply the inclusion of other components rather than the exclusion of any other components.

Terms "first", "second", etc. are provided for discriminating one component from another component and the scope of a right is not limited to the terms. For example, the first component may be named the second component, and vice versa.

FIG. 1 is a flowchart illustrating an electrical stimulation application exemplary method for pneumogastric nerves reflecting degree of symptoms according to biosignal measurement.

In a first embodiment according to the present disclosure, electrical stimulation is applied to a pneumogastric nerve by reflecting the degree of symptom on the basis of brain waves.

The electrical stimulation application method for pneumogastric nerves of this embodiment performs first a reference value setting step.

A reference value is a value that is the reference for determining the current state of a user and, in this embodiment, an FSWR (Fast-to-Slow Wave Ratio) is used as an index reflecting the current state of the symptom of a user. The FSWR for setting a reference value is a value obtained by dividing the power of a fast wave band with a relatively high frequency by the power of a slow wave band with a relatively low frequency in a brain wave measured when electrical stimulation is not applied. In this case, the fast wave band may be a brain wave exceeding 13Hz and the slow wave band may be a brain wave of 13Hz or less. Variation of an FSWR means variation of the state of a user in symptom, and a step of configuring the reference of an FSWR for checking whether a state has changed is the reference value setting step. Since this is for setting a reference for determining the state of a user, a plurality of pieces of FSWR information may be used. In this case, an FSWR may be calculated and an FSWRₛ that is an FSWR reference value may be set by repeatedly measuring brain waves with predetermined intervals to measure information about a normal state rather than information about the state of a specific period. For example, it may be possible to use the results of repeatedly measuring brain waves at the same time of day, and particularly, it may be possible to repeatedly measure brain waves at the same time as the time at which electrical stimulation is scheduled to be applied, and it may be possible to calculate an FSWR and set an FSWRₛ using brain wave information measured at the same time for a week. As a detailed method of setting an FSWRₛ, an FSWRₛ may be set by adding the standard deviation of FSWR values calculated by measuring brain waves a plurality of times to the average of the FSWR values. The reference value setting step is a process that is performed between electrical stimulation is applied, and particularly, it is possible to repeat the reference value setting step to set a new reference value at every predetermined time and it may be possible to set a new reference value with an interval of a week. Such a process of setting an FSWRₛ is performed by a reference value setter. The reference value setter may include a brain wave measurement device that measures brain waves to measure an FSWR and derive a reference value, and a storage device and a calculation device for storing brain wave data and calculating the average and the standard variation of FSWRs.

An electrical stimulation guideline input step is performed after an FSWRₛ that is an FSWR reference value is set.

An electrical stimulation guideline includes information about electrical stimulation for stimulating a pneumogastric nerve. An electrical stimulation guideline may be determined on the basis diagnosis and prescription by a doctor at hospital or may be derived through a separate program or system. An electrical stimulation guideline for stimulating a pneumogastric nerve may include information such as the current, frequency, duty ratio, stimulation time of electrical stimulation. Such an electrical stimulation guideline is determined in accordance with the disease or symptoms of a user, but a new electrical stimulation guideline is not given every time an electrical stimulation application apparatus is used, so there may be no effect of electrical stimulation applied in accordance with an electrical stimulation guideline due to the time difference between the point in time when an electrical stimulation guideline was given and the point in time when electrical stimulation is applied. In order to solve this problem, the present disclosure is characterized by evaluating the current state of a user on the basis of an FSWR reference value obtained in advance by measuring brain waves, and adjusting electrical stimulation by reflecting the current state.

An electrical stimulation guideline including predetermined information about electrical stimulation, as described above, is input through an input unit of the electrical stimulation application apparatus for pneumogastric nerves. As a method of inputting an electrical stimulation guideline, a user may manually input information about electrical stimulation or it may be possible to automatically receive an electrical stimulation guideline through a code (a barcode or a QR code) or an information storage device that includes information about electrical stimulation.

In this embodiment, initial stimulation value setting step is performed before electrical stimulation that is applied to a user is adjusted by reflecting the current state of the user.

Initial stimulation values are values setting an electrical stimulation guideline as electrical stimulation in a range suitable for a user by reflecting sensitivity to electrical stimulation. Sensitivity for electrical stimulation is a concept coming from the fact that people have different senses of pain (pain) or discomfort in response to the same electrical stimulation, and means the degree to which one can easily perceive pain or discomfort in response to electrical stimulation. In the present disclosure, sensitivity to electrical stimulation reflects not only sensitivity depending on the intensity of physical stimulation and sensitivity that human perceives as pain, but also sensitivity to psychological discomfort or anxiety even though pain is not felt. When the sensitivity to electrical stimulation is high, the possibility of feeling pain or discomfort to the same electrical stimulation is high, and particularly, as a current increases, more pain or discomfort is felt.

The present disclosure applies a Stimulation Perception Index (SPI) as an index quantifying sensitivity to electrical stimulation. In the present disclosure, an SPI is calculated by applying demographic markers and/or environmental markers. Demographic markers are markers that statistically quantify sensitivity to electrical stimulation on the basis of biometric information such as age, sex, Body Mass Index (BMI), presence of medication, and presence of disease. For example, the sensitivity to electrical stimulation is higher in children and the elderly than in young adults, higher in women than in men, and lower as the BMI increases. Demographic markers are statistically organized so that this information can be applied to the SPI index. Environmental markers are indictors that statistically represent sensitivity to electrical stimulation on the basis of environmental information such as temperature, humidity, illuminance, and a discomfort index. For example, when temperature or humidity is lower than a specific numerical value, sensitivity to electrical stimulation is high, when illuminance is low, sensitivity to electrical stimulation is high, and sensitivity to electrical stimulation changes, depending on the range of a discomfort index. When sensitivity to electrical stimulation indicated by an SPI index increases, this is the case when it is easy to feel pain or discomfort from electrical stimulation. Accordingly, initial stimulation values are set to decrease the degree of stimulation so that a user does not feel pain or discomfort, and for example, it may be possible to decrease the intensity of a current. However, a value that can provide a predetermined effect is provided for an electrical stimulation guideline, and when only the intensity of a current is decreased, sufficient stimulation is not applied, so the effect by application of electrical stimulation may not be obtained. Accordingly, compensation is needed as much as reducing a current and it is possible to apply a method of increasing a duty ratio in proportion to reduction of a current. The frequency of an electrical stimulation guideline may not be changed, and when the time for which electrical stimulation is applied is increased, discomfort of a user increases, so the application time of electrical stimulation may be maintained. Sensitivity to electrical stimulation is evaluated high only when an SPI is larger than a predetermined value, so it is possible to set initial stimulation values such that a current decreases and a duty ratio increases in proportion to an excess value. On the other hand, when an SPI is a predetermined value or less, it is possible to apply the current and the duty ratio of an electrical stimulation guideline as initial stimulation values. As described above, the process of deriving initial stimulation values from an input electrical stimulation guideline is performed by the initial stimulation value setter, and the initial stimulation value setter may include a DB, etc. for deriving an SPI, may include an input device for receiving age, sex, etc. to apply demographic markers, and may include an BMI directly measurer and apply a BMI. Further, the initial stimulation value setter may include an input device for receiving information to apply environmental markers and may include a thermometer or a hygrometer for directly measuring and reflecting temperature, humidity, etc.

Next, a stimulation value adjustment step is performed.

A stimulation value means electrical stimulation that is applied to a user through an electrode, and, in the present disclosure, an electrical stimulation guideline or initial stimulation values reflecting sensitivity to an electrical stimulation guideline is not applied as it is, and an electrical stimulation guideline or initial stimulation values are adjusted and used to a user with the current state of the user reflected. In detail, an electrical stimulation guideline or initial stimulation values are adjusted by comparing an FSWRₜ calculated by measuring a brain wave of a user with an FSWRₛ set in the previous reference value setting step before electrical stimulation is applied. For example, when an FSWRₜ is larger than an FSWRₛ, it is possible to adjust electrical stimulation that is applied through an electrode. In more detail, when an FSWRₜ is larger than an FSWRₛ, it is evaluated that the state of a user became worse than normal and the duty ratio of electrical stimulation included in an electrical stimulation guideline or initial stimulation values is increased such that the total amount of electrical stimulation that is applied to the user increases. In this case, it is possible to increase the duty ratio of electrical stimulation in proportion to the difference between an FSWRₜ and an FSWRₛ. Adjustment of an electrical stimulation guideline or initial stimulation values is performed by a stimulation value adjuster. The stimulation value adjuster may include a brain wave measurement device that measures brain waves to calculate an FSWR, and a storage device, a calculation device, etc. for storing brain wave data and calculating and comparing an FSWR with a reference value. The stimulation value adjuster can use all or some of components with the reference value setter.

Further, an electrical stimulation step of applying electrical stimulation to an electrode with an electrical stimulation or initial stimulation values adjusted is performed.

According to the above processes, it is possible to reduce the problem that a user feels pain by adjusting an electrical stimulation guideline by reflecting the sensitivity of the user and electrical stimulation is applied to a user with an electrical stimulation guideline or initial stimulation values adjusted by reflecting the current state of the user, so there is an effect of improving the effect by application of electrical stimulation. It is possible to apply electrical stimulation to a user with an electrical stimulation guideline or initial stimulation values adjusted through a controller that controls electrical signals that are applied to an electrode.

In a second embodiment according to the present disclosure, electrical stimulation is applied to a pneumogastric nerve by reflecting the degree of symptom on the basis of heart rate variability.

The electrical stimulation application method for pneumogastric nerves of this embodiment performs first a reference value setting step. Heart rate variability that means the degree of variation of heartbeat means fine variation between one heart cycle and the next heart cycle. Meanwhile, a pulse, though different at measurement positions, is the same as heartbeat or can be used instead of heartbeat in that it reflects the characteristics of heartbeat. Accordingly, pulse variability can be used instead of heart rate variability or used almost the same, and measurement of heart rate variability can be replaced with measurement of pulse variability.

A reference value is a value that is the reference for determining the current state of a user and, in this embodiment, an FSBR (Fast-to-Slow Beat Ratio) is used as an index reflecting the current state of the symptom of a user. An FSBR measured to set a reference value is a value calculated by dividing power of a high frequency band with a relatively high frequency by power of a low frequency band with a relatively low frequency in heart rate variability measured when electrical stimulation is not applied. In this case, the high frequency band may be the range of 0.15Hz or more and 0.4Hz or less and the low frequency band is the range of 0.04Hz or more and less than 0.15Hz. Variation of an FWBR means variation of the state of a user in symptom, and a step of configuring the reference of an FSBR for checking whether a state has changed is the reference value setting step. Since this is for setting a reference for determining the state of a user, a plurality of pieces of FSBR information may be used. In this case, an FSBR may be calculated and an FSBRₛ that is an FSBR reference value may be set by repeatedly measuring heart rate variability with predetermined intervals to measure information about a normal state rather than information about the state of a specific period. For example, it may be possible to use the results of repeatedly measuring heart rate variability at the same time of day, and particularly, it may be possible to repeatedly measure brain waves at the same time as the time at which electrical stimulation is scheduled to be applied, and it may be possible to calculate an FSBR and set an FSBRₛ using heart rate variability information measured at the same time for a week. As a detailed method of setting an FSBR S, an FSBRₛ may be set by adding the standard deviation of FSBR values calculated by measuring heart rate variability a plurality of times to the average of the FSBR values. The reference value setting step is a process that is performed between electrical stimulation is applied, and particularly, it is possible to repeat the reference value setting step to set a new reference value at every predetermined time and it may be possible to set a new reference value with an interval of a week. Such a process of setting an FSBRₛ is performed by a reference value setter. The reference value setter may include an electrocardiogram machine that measures heart rate variability to measure an FSBR and derive a reference value, and a storage device and a calculation device for storing electrocardiogram data and calculating the average and the standard variation of FSBRs.

An electrical stimulation guideline input step is performed after an FSBRₛ that is an FSBR reference value is set.

An electrical stimulation guideline includes information about electrical stimulation for stimulating a pneumogastric nerve. An electrical stimulation guideline may be determined on the basis diagnosis and prescription by a doctor at a hospital or may be derived through a separate program or system. An electrical stimulation guideline for stimulating a pneumogastric nerve may include information such as the current, frequency, duty ratio, stimulation time of electrical stimulation. Such an electrical stimulation guideline is determined in accordance with the disease or symptoms of a user, but a new electrical stimulation guideline is not given every time an electrical stimulation application apparatus is used, so there may be no effect of electrical stimulation applied in accordance with an electrical stimulation guideline due to the time difference between the point in time when an electrical stimulation guideline was given and the point in time when electrical stimulation is applied. In order to solve this problem, the present disclosure is characterized by evaluating the current state of a user on the basis of an FSBR reference value obtained in advance by measuring heart rate variability, and adjusting electrical stimulation by reflecting the current state.

An electrical stimulation guideline including predetermined information about electrical stimulation, as described above, is input through an input unit of the electrical stimulation application apparatus. As a method of inputting an electrical stimulation guideline, a user may manually input information about electrical stimulation or it may be possible to automatically receive an electrical stimulation guideline through a code (a barcode or a QR code) or an information storage device that includes information about electrical stimulation.

In this embodiment, initial stimulation value setting step is performed before electrical stimulation that is applied to a user is adjusted by reflecting the current state of the user.

Initial stimulation values are values setting an electrical stimulation guideline as electrical stimulation in a range suitable for a user by reflecting sensitivity to electrical stimulation. Sensitivity for electrical stimulation is a concept coming from the fact that people have different senses of pain (pain) or discomfort in response to the same electrical stimulation, and means the degree to which one can easily perceive pain or discomfort in response to electrical stimulation. In the present disclosure, sensitivity to electrical stimulation reflects not only sensitivity depending on the intensity of physical stimulation and sensitivity that human perceives as pain, but also sensitivity to psychological discomfort or anxiety even though pain is not felt. When the sensitivity to electrical stimulation is high, the possibility of feeling pain or discomfort to the same electrical stimulation is high, and particularly, as a current increases, more pain or discomfort is felt.

The present disclosure applies a Stimulation Perception Index (SPI) as an index quantifying sensitivity to electrical stimulation. In the present disclosure, an SPI is calculated by applying demographic markers and/or environmental markers. Demographic markers are markers that statistically quantify sensitivity to electrical stimulation on the basis of biometric information such as age, sex, Body Mass Index (BMI), presence of medication, and presence of disease. For example, the sensitivity to electrical stimulation is higher in children and the elderly than in young adults, higher in women than in men, and lower as the BMI increases. Demographic markers are statistically organized so that this information can be applied to the SPI index. Environmental markers are indictors that statistically represent sensitivity to electrical stimulation on the basis of environmental information such as temperature, humidity, illuminance, and a discomfort index. For example, when temperature or humidity is lower than a specific numerical value, sensitivity to electrical stimulation is high, when illuminance is low, sensitivity to electrical stimulation is high, and sensitivity to electrical stimulation changes, depending on the range of a discomfort index. When sensitivity to electrical stimulation indicated by an SPI index increases, this is the case when it is easy to feel pain or discomfort from electrical stimulation. Accordingly, initial stimulation values are set to decrease the degree of stimulation so that a user does not feel pain or discomfort, and for example, it may be possible to decrease the intensity of a current. However, a value that can provide a predetermined effect is provided for an electrical stimulation guideline, and when only the intensity of a current is decreased, sufficient stimulation is not applied, so the effect by application of electrical stimulation may not be obtained. Accordingly, compensation is needed as much as reducing a current and it is possible to apply a method of increasing a duty ratio in proportion to reduction of a current. The frequency of an electrical stimulation guideline may not be changed, and when the time for which electrical stimulation is applied is increased, discomfort of a user increases, so the application time of electrical stimulation may be maintained. Sensitivity to electrical stimulation is evaluated high only when an SPI is larger than a predetermined value, so it is possible to set initial stimulation values such that a current decreases and a duty ratio increases in proportion to an excess value. On the other hand, when an SPI is a predetermined value or less, it is possible to apply the current and the duty ratio of an electrical stimulation guideline as initial stimulation values. As described above, the process of deriving initial stimulation values from an input electrical stimulation guideline is performed by the initial stimulation value setter, and the initial stimulation value setter may include a DB, etc. for deriving an SPI, may include an input device for receiving age, sex, etc. to apply demographic markers, and may include an BMI measurer to directly measure and apply a BMI. Further, the initial stimulation value setter may include an input device for receiving information to apply environmental markers and may include a thermometer or a hygrometer for directly measuring and reflecting temperature, humidity, etc.

Next, a stimulation value adjustment step is performed.

A stimulation value means electrical stimulation that is applied to a user through an electrode, and, in the present disclosure, an electrical stimulation guideline or initial stimulation values reflecting sensitivity to an electrical stimulation guideline is not applied as it is, and an electrical stimulation guideline or initial stimulation values are adjusted and used to a user with the current state of the user reflected. In detail, an electrical stimulation guideline or initial stimulation values are adjusted by comparing an FSBRₜ calculated by measuring heart rate variability of a user with an FSBRₛ set in the previous reference value setting step before electrical stimulation is applied. For example, when an FSBRₜ is larger than an FSBRₛ, it is possible to adjust electrical stimulation that is applied through an electrode. In more detail, when an FSBRₜ is larger than an FSBRₛ, it is evaluated that the state of a user became worse than normal and the duty ratio of electrical stimulation included in an electrical stimulation guideline or initial stimulation values is increased such that the total amount of electrical stimulation that is applied to the user increases. In this case, it is possible to increase the duty ratio of electrical stimulation in proportion to the difference between an FSWRₜ and an FSWRₛ. Adjustment of an electrical stimulation guideline or initial stimulation values is performed by a stimulation value adjuster. The stimulation value adjuster may include an electrocardiogram machine that measures heart rate variability to calculate an FSBR, and a storage device, a calculation device, etc. for storing electrocardiogram data and calculating and comparing an FSBR with a reference value. The stimulation value adjuster can use all or some of components with the reference value setter.

Further, an electrical stimulation step of applying electrical stimulation to an electrode with an electrical stimulation or initial stimulation values adjusted is performed.

According to the above processes, it is possible to reduce the problem that a user feels pain by adjusting an electrical stimulation guideline by reflecting the sensitivity of the user and electrical stimulation is applied to a user with an electrical stimulation guideline or initial stimulation values adjusted by reflecting the current state of the user, so there is an effect of improving the effect by application of electrical stimulation. It is possible to apply electrical stimulation to a user with an electrical stimulation guideline or initial stimulation values adjusted through a controller that controls electrical signals that are applied to an electrode.

## Claims

1. An electrical stimulation application apparatus for pneumogastric nerves, the electrical stimulation application apparatus comprising:
an electrode configured to apply electrical stimulation in contact with the skin of a human body;
an input unit configured to receive an electrical stimulation guideline comprising numerical information about electrical stimulation;
a reference value setter configured to set a reference value for a biosignal of a user from the biosignal measured a plurality of times over predetermined intervals without electrical stimulation applied; and
a stimulation value adjuster configured to adjust the electrical stimulation guideline by comparing a value calculated by measuring the biosignal of the user with the reference value before applying electrical stimulation,
wherein the biosignal that is measured is a brain wave,
the reference value setter calculates an FSWR from each of brain wave information of the user measured a plurality of times over the predetermined intervals and sets an FSWRs that is an FSWR reference value,
the stimulation value adjuster adjusts the electrical stimulation guideline by comparing an FSWRt calculated by measuring a brain wave of the user with the FSWRs before applying electrical stimulation, and
the FSWR is an index reflecting a state of a symptom of the user and is calculated by dividing power of a fast wave band with a relatively high frequency by power of a slow wave band with a relatively low frequency in the measured brain wave,
wherein the fast wave band is a brain wave exceeding 13Hz and the slow wave band is a brain wave of 13Hz or less.

2. The electrical stimulation application apparatus of claim 1, wherein the FSWRs is obtained by adding standard deviation of FSWR values calculated by measuring a brain wave a plurality of times to an average of the FSWR values.

3. The electrical stimulation application apparatus of claim 1, wherein when the FSWRt is larger than the FSWRs, the stimulation value adjuster adjusts electrical stimulation that is applied through the electrodeor a duty ratio of electrical stimulation is increased.

4. The electrical stimulation application apparatus of claim 3, wherein the duty ratio is increased in proportion to a difference between the FSWRt and the FSWRs.

5. The electrical stimulation application apparatus of claim 1, further comprising an initial stimulation value setter configured to derive initial stimulation values adjusting numerical values comprised in the electrical stimulation guideline by applying an SPI calculated by applying one or more of demographic markers and environmental markers;
wherein the SPI is an index quantifying sensitivity to electrical stimulation, the demographic markers are markers statistically reflecting sensitivity to electrical stimulation, and the environmental markers are markers reflecting environmental factors influencing sensitivity to electrical stimulation; and
the stimulation value adjuster adjusts the initial stimulation values derived by the initial stimulation value setter.

6. The electrical stimulation application apparatus of claim 5, wherein the demographic markers comprise one or more pieces of information of age, sex, BMI, presence of medication, and presence of disease or wherein the environmental markers comprise one or more pieces of information of temperature, humidity, illuminance, and a discomfort index.

## Patentansprüche

1. Eine elektrische Stimulationsanwendungsvorrichtung für pneumogastrische Nerven, wobei die elektrische Stimulationsanwendungsvorrichtung umfasst:
eine Elektrode, die so konfiguriert ist, dass sie elektrische Stimulation in Kontakt mit der Haut eines menschlichen Körpers anwendet;
eine Eingabeeinheit, die so konfiguriert ist, dass sie eine Richtlinie zur elektrischen Stimulation empfängt, die numerische Informationen über die elektrische Stimulation umfasst;
einen Referenzwertgeber, der so konfiguriert ist, dass er einen Referenzwert für ein Biosignal eines Benutzers aus dem Biosignal festlegt, das mehrfach über vorbestimmte Intervalle ohne angelegte elektrische Stimulation gemessen wurde; und
einen Stimulationswertanpasser, der so konfiguriert ist, dass er die Richtlinie zur elektrischen Stimulation anpasst, indem ein Wert, der durch Messung des Biosignals des Benutzers berechnet wurde, mit dem Referenzwert vor der Anwendung der elektrischen Stimulation verglichen wird,
wobei das gemessene Biosignal eine Gehirnwelle ist,
der Referenzwertgeber aus jeder der mehrfach über die vorbestimmten Intervalle gemessenen Gehirnwelleninformationen des Benutzers einen FSWR berechnet und einen FSWRs als FSWR-Referenzwert festlegt,
der Stimulationswertanpasser die Richtlinie zur elektrischen Stimulation anpasst, indem ein FSWRt, der durch Messung einer Gehirnwelle des Benutzers berechnet wird, vor der Anwendung der elektrischen Stimulation mit dem FSWRs verglichen wird, und
der FSWR ein Index ist, der den Zustand eines Symptoms des Benutzers widerspiegelt und berechnet wird, indem die Leistung eines schnellen Wellenbands mit relativ hoher Frequenz durch die Leistung eines langsamen Wellenbands mit relativ niedriger Frequenz in der gemessenen Gehirnwelle geteilt wird,
wobei das schnelle Wellenband eine Gehirnwelle über 13 Hz ist und das langsame Wellenband eine Gehirnwelle von 13 Hz oder weniger ist.

2. Die elektrische Stimulationsanwendungsvorrichtung nach Anspruch 1, wobei der FSWRs erhalten wird, indem die Standardabweichung der FSWR-Werte, die durch mehrfaches Messen einer Gehirnwelle berechnet wurden, zu dem Mittelwert der FSWR-Werte addiert wird.

3. Die elektrische Stimulationsanwendungsvorrichtung nach Anspruch 1, wobei der Stimulationswertanpasser die elektrische Stimulation, die über die Elektrode angewendet wird, anpasst oder das Tastverhältnis der elektrischen Stimulation erhöht, wenn der FSWRt größer als der FSWRs ist.

4. Die elektrische Stimulationsanwendungsvorrichtung nach Anspruch 3, wobei das Tastverhältnis proportional zu dem Unterschied zwischen FSWRt und FSWRs erhöht wird.

5. Die elektrische Stimulationsanwendungsvorrichtung nach Anspruch 1, die ferner einen Einstellwertgeber für die Anfangsstimulation umfasst, der so konfiguriert ist, dass er Anfangsstimulationswerte ableitet, indem numerische Werte, die in der Richtlinie für elektrische Stimulation enthalten sind, durch Anwendung eines SPI angepasst werden, der durch Anwendung eines oder mehrerer demografischer Marker und Umweltmarker berechnet wird;
wobei der SPI ein Index ist, der die Empfindlichkeit gegenüber elektrischer Stimulation quantifiziert, die demografischen Marker Marker sind, die statistisch die Empfindlichkeit gegenüber elektrischer Stimulation widerspiegeln, und die Umweltmarker Marker sind, die Umweltfaktoren widerspiegeln, die die Empfindlichkeit gegenüber elektrischer Stimulation beeinflussen; und
der Stimulationswertanpasser die vom Einstellwertgeber für die Anfangsstimulation abgeleiteten Anfangsstimulationswerte anpasst.

6. Die elektrische Stimulationsanwendungsvorrichtung nach Anspruch 5, wobei die demografischen Marker eine oder mehrere Informationen über Alter, Geschlecht, BMI, Einnahme von Medikamenten und Vorliegen von Krankheiten umfassen, oder wobei die Umweltmarker eine oder mehrere Informationen über Temperatur, Luftfeuchtigkeit, Beleuchtungsstärke und einen Unbehaglichkeitsindex umfassen.

## Revendications

1. Un appareil d'application de stimulation électrique pour nerfs pneumogastriques, l'appareil d'application de stimulation électrique comprenant :
une électrode configurée pour appliquer une stimulation électrique en contact avec la peau d'un corps humain ;
une unité d'entrée configurée pour recevoir une directive de stimulation électrique
comprenant des informations numériques sur la stimulation électrique ;
un définisseur de valeur de référence configuré pour établir une valeur de référence pour un biosignal de l'utilisateur à partir du biosignal mesuré plusieurs fois à des intervalles prédéterminés sans stimulation électrique appliquée ; et
un régulateur de valeur de stimulation configuré pour ajuster la directive de stimulation électrique en comparant une valeur calculée par la mesure du biosignal de l'utilisateur avec la valeur de référence avant l'application de la stimulation électrique,
dans lequel le biosignal mesuré est une onde cérébrale,
le définisseur de valeur de référence calcule un FSWR à partir de chacune des informations d'ondes cérébrales de l'utilisateur mesurées plusieurs fois sur les intervalles prédéterminés et définit un FSWRs comme valeur de référence FSWR,
le régulateur de valeur de stimulation ajuste la directive de stimulation électrique en comparant un FSWRt calculé en mesurant une onde cérébrale de l'utilisateur avec le FSWRs avant l'application de la stimulation électrique,
et
le FSWR est un indice reflétant l'état d'un symptôme de l'utilisateur, calculé en divisant la puissance d'une bande d'ondes rapides à fréquence relativement élevée par la puissance d'une bande d'ondes lentes à fréquence relativement basse dans l'onde cérébrale mesurée,
dans lequel la bande d'ondes rapides est une onde cérébrale supérieure à 13 Hz et la bande d'ondes lentes est une onde cérébrale de 13 Hz ou moins.

2. L'appareil d'application de stimulation électrique selon la revendication 1, dans lequel le FSWRs est obtenu en ajoutant l'écart type des valeurs FSWR calculées en mesurant plusieurs fois l'onde cérébrale à la moyenne des valeurs FSWR.

3. L'appareil d'application de stimulation électrique selon la revendication 1, dans lequel le régulateur de valeur de stimulation ajuste la stimulation électrique appliquée par l'électrode ou le rapport cyclique de la stimulation électrique est augmenté lorsque le FSWRt est supérieur au FSWRs.

4. L'appareil d'application de stimulation électrique selon la revendication 3, dans lequel le rapport cyclique est augmenté proportionnellement à la différence entre le FSWRt et le FSWRs.

5. L'appareil d'application de stimulation électrique selon la revendication 1, comprenant en outre un définisseur de valeurs de stimulation initiale, configuré pour dériver des valeurs de stimulation initiales en ajustant les valeurs numériques comprises dans la directive de stimulation électrique par application d'un SPI calculé en appliquant un ou plusieurs marqueurs démographiques et environnementaux ;
dans lequel le SPI est un indice quantifiant la sensibilité à la stimulation électrique, les marqueurs démographiques sont des marqueurs reflétant statistiquement la sensibilité à la stimulation électrique, et les marqueurs environnementaux sont des marqueurs reflétant les facteurs environnementaux influençant la sensibilité à la stimulation électrique ; et
le régulateur de valeur de stimulation ajuste les valeurs de stimulation initiales dérivées par le définisseur de valeurs de stimulation initiale.

6. L'appareil d'application de stimulation électrique selon la revendication 5, dans lequel les marqueurs démographiques comprennent une ou plusieurs informations sur l'âge, le sexe, l'IMC, la prise de médicaments et la présence de maladies, ou dans lequel les marqueurs environnementaux comprennent une ou plusieurs informations sur la température, l'humidité, l'illumination et un indice d'inconfort.
